# FASCICULE DE BREVET EUROPEEN

(11) **EP 2 882 840 B1**
(45) Date de publication et mention de la délivrance du brevet: **03.03.2021**
(21) Numéro de dépôt: 13773279.8
(22) Date de dépôt: 13.08.2013
(51) Int. Cl.: C12M 1/107, C12M 1/00, C12M 1/16

(54) **PROCÉDÉ ET DISPOSITIF DE MÉTHANISATION CONTINUE EN VOIE SÈCHE**
VERFAHREN UND VORRICHTUNG ZUR KONTINUIERLICHEN TROCKENEN METAHNISIERUNG
METHOD AND DEVICE FOR CONTINUOUS DRY METHANISATION

(30) Priorité: 13.08.2012 FR 1257782; 20.12.2012 FR 1262512; 20.12.2012 FR 1262513; 20.12.2012 FR 1262514
(43) Date de publication de la demande: 17.06.2015
(73) Titulaire: Arkolia Energies, 34130 Mudaison (FR); Bonhomme, Michel, 74150 Rumilly (FR)
(72) Inventeur: BONHOMME, Michel, 74150 Rumilly (FR)
(74) Mandataire: Cornuejols, Georges
(86) Numéro de dépôt international: PCT/FR2013/051938
(87) Numéro de publication internationale: WO 2014/027165

(56) Documents cités:
- CN-U- 202 022 920
- FR-A- 874 378
- FR-A1- 2 794 472

## Description

### DOMAINE TECHNIQUE DE L'INVENTION

La présente invention concerne un procédé et un dispositif de méthanisation continue en voie sèche. Elle s'applique, en particulier à la méthanisation continue en voie sèche dans des compartiments à milieux physiques et biochimiques différents, par brassage sectoriel, à fort débit modulable de gaz.

### ÉTAT DE LA TECHNIQUE

La fermentation anaérobie des effluents et déchets vise à réduire la matière organique et à produire de l'énergie sous forme de biogaz. On connaît essentiellement la méthanisation des boues en phase liquide issue du traitement des effluents des stations d'épuration des eaux urbaines, la méthanisation de la fraction fermentescibles des déchets ménagers et la méthanisation à la ferme des fumiers et autres déchets agricoles. On trouve également la méthanisation des effluents de l'industrie agro-alimentaires en phase liquide à faible concentration de matière sèche. On a vu se développer des unités collectives de co-digestion intégrant le traitement de substrats différents d'origines différentes (urbaines, industrielle et agricole).

La fermentation se fait en continu (chargement et déchargement continu ou séquentiel de la matière sans vidange du digesteur) ou en discontinu (chargement de la matière ensemencée par une matière déjà fermentée avec vidange du digesteur et rechargement par un substrat nouveau).

L'homme de l'art qui exploite des installations de méthanisation de substrats solides rencontre les limites des systèmes courants qui fonctionnent en méthanisation avec chargement continu, aux seules fins d'agiter une matière en fermentation dans une cuve à géométrie variable.

Les procédés d'agitation mécanique de la matière, pâles mobiles, vis sans fin, cylindre mobile ou agitation par un gaz surpressé ou comprimé sont connus.

Par exemple le document CN 202 022 920 décrit la digestion anaérobie d'ordures ménagères dans un fermenteur dans lequelle le gaz produit est re-injecté du haut du réservoir au moyen de tuyaux descendant verticalement jusqu'à près du fond pour agiter la matière.

L'agitation peut être en continu à faible pression ou séquentiel à forte pression, tel que présenté dans le document FR 2 794 472, dans des tuyauterie ou des injecteurs de faibles diamètres recevant des vitesses et des débits de gaz peu important, du fait de la dispersion sur de multiples injecteurs ou rampes d'injection, avec de multiples ouvertures d'injection. De même un digesteur à plusieurs compartiments est connu. Mais les procédés connus d'agitation, dans au moins deux compartiments, ne permettent pas l'écoulement d'une matière épaisse proche des seuils de cisaillement et ne sont pas adaptés pour permettre le brassage de la matière dans la totalité d'un volume donné et ne peuvent permettre une maîtrise de la fermentation permettant une dégradation importante de la matière en fermentation dans des temps de séjours courts et sans dilution importante de la matière.

Les systèmes de brassage au gaz par injection du gaz depuis le fond de cuves nécessitent une structure en génie civil particulièrement importante pour accéder aux conduits de gaz débouchant au fond de la cuve de fermentation. Ces conduits d'injection du gaz sous pression par intermittence ne supportent pas d'éventuel reflux de matière dans la tuyauterie de gaz, pouvant boucher les injecteurs. Le bout de l'injecteur est forcément étroit ce qui engendre un jet de gaz étroit, ascendant qui ne peut qu'avoir un impact limité sur le brassage d'une matière épaisse, ce qui crée lors des injections de gaz des passages préférentiels dans la matière limitant le brassage et le retournement de celle-ci, notamment pour la matière en fond de cuve et rendant ainsi difficile la remise en suspension des éléments lourds qui risquent de s'accumuler au fond du fermenteur. L'insuffisance du brassage rend ainsi l'écoulement de la matière plus difficile avec des séparations de phases créant des zones mortes et des vitesses de fermentation différentes dans le fermenteur.

On note aussi que les phases de la fermentation ont pu être suffisamment maîtrisées par des effets de dilution importants de la matière pour favoriser son écoulement ce qui a pour conséquence également, de par la nature du brassage, le plus souvent mécanique ou par brassage au gaz, de conduire les fermentations dans des fermenteurs infiniment mélangés. Les temps de séjours de la matière sont alors aléatoires. Les effluents liquides résiduels en fin de fermentation sont importants. Pour certains types d'effluents, le manque de matière sèche est un facteur limitant de la fermentation par manque de support bactérien. Ce manque de matière sèche nécessite l'introduction de supports minéraux afin de fixer les populations bactériennes qui ont besoin de support.

### OBJET DE L'INVENTION

La présente invention vise à remédier à tout ou partie de ces inconvénients.

A cet effet, la présente invention vise, selon un premier aspect, un procédé de méthanisation continue en voie sèche selon la revendication 1.

Préférentiellement, la matière épaisse comporte au moins 22 % de matière sèche.

Grâce à ces dispositions, l'ensemble de la matière épaisse en fermentation est brassée, y compris les parties qui ont tendance à sédimenter.

Dans des modes de réalisation, on introduit la matière épaisse dans un premier compartiment et, après hydrolyse dans le premier compartiment, on fait passer la matière épaisse hydrolysée dans un deuxième compartiment où a lieu la méthanogénèse.

Grâce à ces dispositions, on constitue deux milieux favorables aux différentes phases de la fermentation et on réduit, en conséquence, les dimensions du fermenteur et la durée de la fermentation.

Dans des modes de réalisation, l'étape de passage du premier au deuxième compartiment est effectuée par le fond de la cuve, les atmosphères des compartiments n'étant ainsi pas en contact.

Grâce à des dispositions, les atmosphères des différents compartiments peuvent rester favorables aux réactions en cours dans ces compartiments, par leur composition et/ou par leur chaleur.

Dans des modes de réalisation, au cours de l'injection de gaz, on introduit du gaz issu de la fermentation de la matière épaisse.

Dans des modes de réalisation, au cours de l'étape d'injection de gaz, on injecte du gaz dans au moins deux cheminées proches.

On réalise ainsi des fronts de brassage qui sont favorable au déplacement en translation de la matière en cours de fermentation.

Dans des modes de réalisation, le procédé objet de la présente invention comporte une étape de mesure de viscosité de la matière épaisse, dans lequel, au cours de l'étape d'injection de gaz, la quantité de gaz, son débit et/ou sa vitesse sont fonction de la viscosité mesurée.

On adapte ainsi la quantité de gaz injecté et/ou le brassage de la matière épaisse à son état réel dans le fermenteur.

Dans des modes de réalisation, au cours de l'étape de mesure de viscosité de la matière épaisse, on mesure la résistance de la matière épaisse à l'injection de gaz lors de l'injection de gaz.

Dans des modes de réalisation, au cours de l'étape de mesure de viscosité de la matière épaisse, on mesure une durée d'une baisse de pression prédéterminée du gaz injecté.

Grâce à ces dispositions, c'est l'état réel de la matière épaisse qui est mesuré directement.

Dans des modes de réalisation, le fermenteur comporte une pluralité de cheminées, et, au cours de l'étape d'injection de gaz, on injecte du gaz successivement dans les cheminées.

Dans des modes de réalisation, au cours de l'étape d'injection de gaz, on injecte du gaz successivement dans les cheminées allant de l'amont vers l'aval du chemin suivi par la matière épaisse.

Grâce à ces dispositions, on favorise le déplacement de la matière épaisse le long de la cuve du fermenteur rendue possible par l'homogénéisation d'une matière suffisamment hydrolysée

Dans des modes de réalisation, le procédé objet de la présente invention comporte une étape de prélèvement de matière épaisse sur le chemin suivi par la matière épaisse et une étape d'injection de la matière épaisse en amont du point de prélèvement.

On peut ainsi favoriser les différentes phases de la méthanisation, en ajustant le pH et/ou les colonies bactériennes des différents secteurs de la cuve.

Dans des modes de réalisation, au cours de l'étape d'injection de gaz, on injecte, dans la matière épaisse, un gaz sous une pression au moins cinq fois, et notamment cinq à dix fois supérieure à la pression de la hauteur de la colonne de matière autour de la cheminée, et au minimum 4 bar relatif, en fonction de la viscosité.

On provoque, ainsi, un brassage important.

Dans des modes de réalisation, le procédé objet de la présente invention comporte alternativement :
- une étape d'injection du gaz dans la matière épaisse et
- une étape de mise en compression de biogaz dans un caisson de stockage en pression.

On laisse, ainsi, la matière en fermentation reposer entre deux injections de gaz et on peut mettre en œuvre un compresseur de petite dimension.

Dans des modes de réalisation, le rapport cyclique entre la durée d'injection de gaz et la durée de mise en compression est inférieur à un trentième.

Selon un deuxième aspect, la présente invention vise un dispositif de méthanisation continue en voie sèche selon la revendication 9.

Les avantages, buts et caractéristiques particulières de ce dispositif étant similaires à ceux du procédé objet de la présente invention, ils ne sont pas rappelés ici.

### BRÈVE DESCRIPTION DES FIGURES

D'autres avantages, buts et caractéristiques de la présente invention ressortiront de la description qui va suivre, faite dans un but explicatif et nullement limitatif en regard des dessins annexés, dans lesquels :
- la figure 1 représente schématiquement en coupe vue de dessus, un premier mode de réalisation particulier du dispositif de fermentation objet de la présente invention,
- la figure 2 représente schématiquement, en coupe longitudinale, le premier mode de réalisation particulier du dispositif de fermentation objet de la présente invention,
- la figure 3 représente schématiquement, en coupe transversale, le premier mode de réalisation du dispositif de fermentation objet de la présente invention,
- la figure 4 représente schématiquement, en vue de dessus, un deuxième mode de réalisation du dispositif de fermentation objet de la présente invention,
- la figure 5 représente schématiquement, en vue de dessus, un troisième mode de réalisation avec le premier compartiment intégré dans le deuxième compartiment du dispositif de fermentation objet de la présente invention,
- la figure 6 représente schématiquement, en coupe longitudinale, un quatrième mode de réalisation du dispositif de fermentation objet de la présente invention,
- la figure 7 représente schématiquement, en coupe longitudinale, des flux dans des modes de réalisation particuliers du dispositif de fermentation objet de la présente invention,
- la figure 8 représente schématiquement, en coupe transversale, des flux dans des modes de réalisation particuliers du dispositif de brassage, objet de la présente invention,
- la figure 9 représente schématiquement, en vue de dessous ou de dessus des flux gaz-matière de secteur en secteur dans des modes de réalisation particulier du dispositif de fermentation objet de la présente invention et
- la figure 10 représente schématiquement, en vue de dessous ou de dessus des flux gaz-matière de secteur en secteur dans des modes de réalisation particulier du dispositif de fermentation objet de la présente invention.

### DESCRIPTION D'EXEMPLES DE RÉALISATION DE L'INVENTION

Après avoir noté que les figures ne sont pas à l'échelle, et avant de décrire les figures plus en détails, on donne, ci-après, une description de caractéristiques de modes de réalisation particuliers du procédé et du dispositif objets de la présente invention.

La méthanisation continue en matière épaisse s'effectue dans une enceinte compartimentée à deux niveaux de température et de pH, reposant sur un brassage modulable par secteur, en fonction de la viscosité de la matière en fermentation, par l'injection de gaz à des vitesses et des débits élevés, dans plusieurs cheminées. La configuration de celles-ci crée un mouvement convectif large partant du fond du fermenteur, entraînant la matière sur une large surface.

L'agitation d'une matière épaisse est préférentiellement sectorielle. Le brassage d'une matière proche des seuils d'écoulement par l'injection de gaz en pression présente des limites. Les limites rencontrées dans l'art antérieur sont de deux sortes selon que l'on dilue les substrats ou que l'on fonctionne en voie dite sèche ou matière épaisse :
- la nécessité de diluer très largement les substrats secs de 25% de matière sèche à 8%, 10% de matière sèche nécessite l'apport d'un liquide complémentaire qui est pris le plus souvent sur la partie liquide en excès en sortie de fermenteur. La re-circulation des « jus » excédentaires pour diluer les substrats entrants entraîne nécessairement des phénomènes d'inhibition de la fermentation par la concentration d'azote minéralisé par exemple basifiant, c'est-à-dire augmentant le pH, progressivement le milieu de fermentation et limitant ainsi l'hydrolyse et l'acidogénèse.
- la fermentation sur des substrats secs peu dilués ou pas dilués dans une fermentation en continu, à des taux de matière sèche élevés rencontre d'autres limites qui résident essentiellement dans la maîtrise de la mécanique des fluides.

On constate, dans l'art antérieur, les limites du brassage d'une matière épaisse et des écoulements de celle-ci, avec une incidence forte sur les processus de fermentation. La première difficulté est d'agiter une matière épaisse de bas en haut sur la hauteur et sur un large secteur de matière, L'agitation ou brassage par du gaz doit être suffisamment puissante pour brasser une matière épaisse dans un volume et un secteur déterminé. La particularité d'un flux gazeux, partant forcément d'un point bas, est de s'élargir dans son ascension et de laisser par conséquent la partie la plus basse de la matière à brasser en dehors du mouvement d'agitation.

Ceci engendre des zones non agitées en partie basse des flux gazeux et sur la périphérie des fermenteurs à cause de la perte de charge due aux frottements de la matière se heurtant aux parois. On rencontre ainsi, sur le fond des fermenteurs, sur une épaisseur plus ou moins importante, des sédiments d'éléments plus lourds et l'accumulation de matière non agitée notamment dans l'espace entre deux jets de gaz et à la périphérie des fermenteurs dans les zones proches des parois.

Les limites rencontrées par tous les systèmes d'agitation au gaz par des tuyauteries, injecteurs en fond de cuve ou rampes d'agitation pour le brassage en infiniment mélangé ou en matière épaisse ont des conséquences sur le fonctionnement biochimique et biologique des fermenteurs de l'art antérieur.

Ces zones « mortes » d'accumulation de sédiments se créent et s'amplifient avec le temps. Le contact solide-liquide nécessaire à l'activité bactérienne est rendu difficile. Ce qui perturbe les équilibres bactériens. Sans l'hydrolyse, la viscosité de la matière restant très élevée rend le passage d'un compartiment à l'autre difficile.

Le fonctionnement avec une matière épaisse, donc avec un taux élevé de matière sèche dans le fermenteur ne peut avoir lieu qu'avec une matière qui s'hydrolyse, en milieu acide, permettant les échanges solides-liquides, fortement agités et à une température suffisamment élevée pour diminuer la viscosité.

Nous connaissons l'impact important de la température associée au pH, qui modifie la viscosité et la structure de la matière organique nécessaire au brassage et à l'homogénéisation, et qui influe également sur un développement sélectif de bactéries et sur le processus de dégradation et de transformation de la matière.

C'est ce qui invite à tendre vers des températures hyper-thermophiles pour favoriser l'hydrolyse et l'acidogénèse, tout en prenant en considération la fragilité des bactéries méthanogènes à des températures hyper-thermophiles et à des pH acides.

Le même niveau de température et de pH dans les phases d'hydrolyse, acidogène, d'une part, et de méthanogénèse, d'autre part, limite la fermentation en continue en matière épaisse.

Nous insistons sur la nécessaire interaction, pour la méthanisation en continu en matière épaisse, entre les dispositifs physiques reliés à la mécanique des fluides et à la rhéologie et la biochimie de la fermentation induisant la sélectivité et le développement des populations bactériennes ou des phénomènes d'inhibitions.

L'originalité de modes de réalisation de la présente invention est de pouvoir fonctionner en continu avec une matière épaisse, ou autrement dit, en voie sèche, dans deux compartiments ayant des pH et des niveaux de température différents.

Le terme de « voie sèche », couramment utilisé, est en fait, inapproprié car la matière même très épaisse avec un taux de matière sèche élevé comporte une partie humide, présente dans les cellules de la matière à fermenter, qui est rapidement libérée par l'activité enzymatique et l'élévation de la température du milieu. Cette partie liquide, extérieure à la structure solide du substrat, est nécessaire aux échanges de métabolites qui se dissolvent dans la partie humide pour être accessible aux bactéries. La définition de la matière épaisse mérite donc d'être approfondie. Avant l'entrer dans le fermenteur la matière se présente une forme solide, pelletable, c'est-à-dire déplaçable avec une pelleteuse, qui ne s'écoule pas, seul un jus liquide peut éventuellement s'extraire de la masse et couler. On a à faire à un fluide non newtonien que l'on peut caractériser comme un fluide à seuil, proche du seuil de cisaillement, élastique et visqueux. Lorsque le substrat est trop solide - par exemple plus de 25% de matière sèche pour un fumier ou plus de 30% de matière sèche pour la partie fermentescible des déchets ménagers, il convient de le diluer légèrement avec un effluent liquide, de l'eau ou un liquide dérivant du traitement aval du digestat, liquide recueilli par pressage, évaporation avec production de distillat liquide, ou autre procédé de séparation de la phase solide et de la phase liquide.

La matière, après une éventuelle légère dilution avant d'entrer dans le fermenteur, est introduite par une cheminée, ou tout autre dispositif d'introduction tel que vis sans fin, dans le fermenteur pour être mélangée avec la matière en fermentation en phase d'hydrolyse, présente dans le premier secteur du premier compartiment, fonctionnant à un niveau de température hyper-thermophile (65°C), voire thermophile (55°C). Le brassage d'une matière très épaisse se mélangeant avec une matière déjà présente dans la première partie du fermenteur et ayant subi une hydrolyse thermo-enzymatique est utile au procédé. La viscosité de la matière se modifie sous l'influence de l'hydrolyse thermo-enzymatique (conjonction de la température et de l'action enzymatique des bactéries).

Le système de brassage est utile à l'ensemble du fonctionnement du procédé. Notamment le dispositif permet d'homogénéiser des substrats différents avec des éléments solides comme, par exemple, les fibres de pailles et des éléments plus liquide composés d'éléments en suspension ou soluble, tels que des lisiers et des éléments plus lourds, graviers, cailloux. L'homogénéité est favorable à l'écoulement d'une matière très épaisse dans le premier compartiment, matière épaisse qui se fluidifie avec l'hydrolyse, puis dans les différentes phases de la fermentation pour demeurer néanmoins épaisse. L'homogénéisation et la fluidification dans le premier compartiment sont favorables au passage à travers une paroi de séparation entre deux compartiments. L'efficacité du brassage est également nécessaire pour l'homogénéisation des températures différentes dans les deux compartiments avec des substrats épais ayant une faible diffusivité thermique. Dans des modes de réalisation, on extrait de la matière en fermentation en différents points du fermenteur pour la réintroduire en d'autres points du fermenteur par les cheminées afin d'inoculer un secteur par des colonies bactériennes venant d'un autre secteur. De même, par la réintroduction de substrat en fermentation d'un secteur à un autre secteur, on peut équilibrer la biochimie du second secteur par acidification ou basification.

La présente invention met en œuvre, dans des modes de réalisation :
- une maîtrise du brassage modulable d'une matière épaisse à fort taux de matière sèche, de 17% à 30% de matière sèche, secteur par secteur, selon la viscosité évolutive du substrat en fermentation, dans un fermenteur compartimenté, et
- une progression régulière et l'écoulement d'un substrat épais et homogène nécessaires à l'équilibre des phases d'hydrolyse, acidogène et méthanogène et
- une interaction entre les modalités du brassage d'une matière épaisse et la maîtrise et le pilotage des phases de la méthanisation en continu en matière épaisse.

Le dispositif de fermentation comporte une enceinte de fermentation fermée comportant au moins deux compartiments et un ensemble de cheminées de diamètre suffisamment large, entre 80 mm et 150 mm par exemple, réparties dans l'ensemble du fermenteur et déterminant des secteurs d'agitation du fermenteur. Chaque cheminée part du plafond de l'enceinte et plonge dans l'enceinte de fermentation jusqu'à proximité du fond de l'enceinte, préférentiellement à moins de 15 cm du fond.

Préférentiellement, les cheminées d'injection de gaz sont configurées pour s'étendre depuis le plafond de l'enceinte, ou cuve, sur au moins 97 % de la hauteur séparant le plafond du fond de l'enceinte et, préférentiellement au moins 98 %.

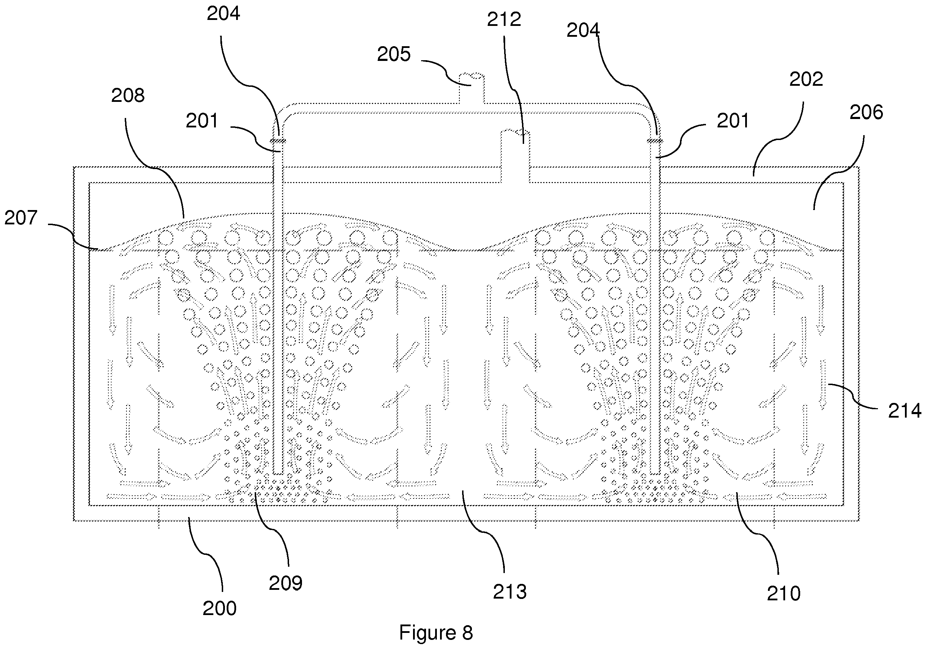

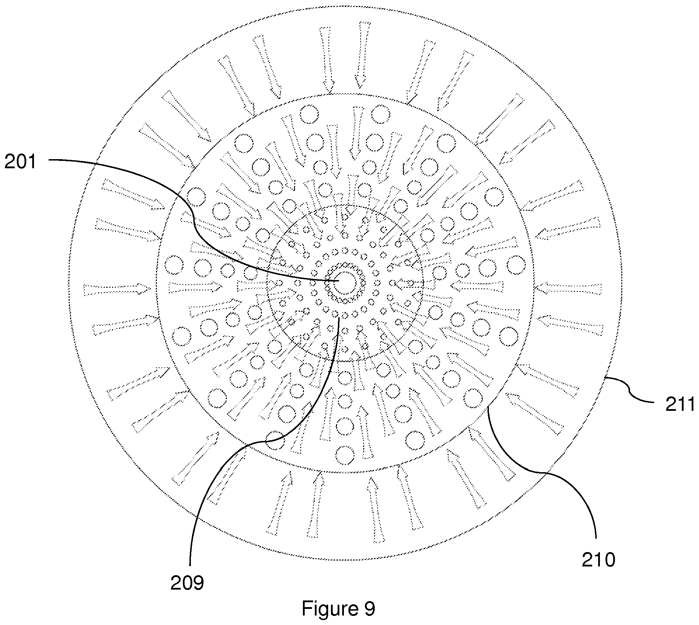

Préférentiellement, le brassage d'une matière épaisse est effectué par secteur en injectant, dans au moins deux cheminées d'un diamètre suffisant, une quantité importante de gaz, préférentiellement du biogaz issu de la méthanisation, à un débit entre 2000 m³/heure et 6000 m³/heure, pendant une durée courte, par exemple entre 10 et 15 secondes. L'injection de gaz, qui se fait à une vitesse et un débit important, de haut en bas en partant du plafond jusqu'à une faible distance du plancher, se heurte au plancher, créant ainsi un cône de gaz sur le plancher et une large colonne de gaz montante, s'élargissant dans son ascension, jusqu'au point haut de la matière, engendrant un gonflement 208 (figure 8) suivi de vagues importantes, le cône de gaz aspirant notamment par le bas, en fond de fermenteur, la matière qui entre dans un mouvement convectif de 1,5 m à 2 m de rayon, dans une zone environnant la colonne de gaz montante et créant ainsi un balayage du fond dans un rayon équivalent, 210 et 211 (figure 9). Le rayon de la partie tronquée du cône de gaz, est fonction de la vitesse, du débit de gaz et de la viscosité de la matière.

Le brassage d'une matière épaisse sur toute la hauteur de matière, dans un secteur déterminé, est rendu possible grâce à un compresseur, un caisson de gaz à haute pression, les cheminées partant du plafond pour déboucher tout près du plancher, recevant du gaz par injection de gaz de haut en bas, sur une courte durée.

Les cheminées sont, le plus souvent, disposées au minimum en couple pour prendre en considération la contrainte de cisaillement engendrée par les frottements de la matière en fermentation sur les deux parois canalisant la matière dans son évolution de type piston, parois délimitant le flux de matière s'écoulant d'un secteur à l'autre.

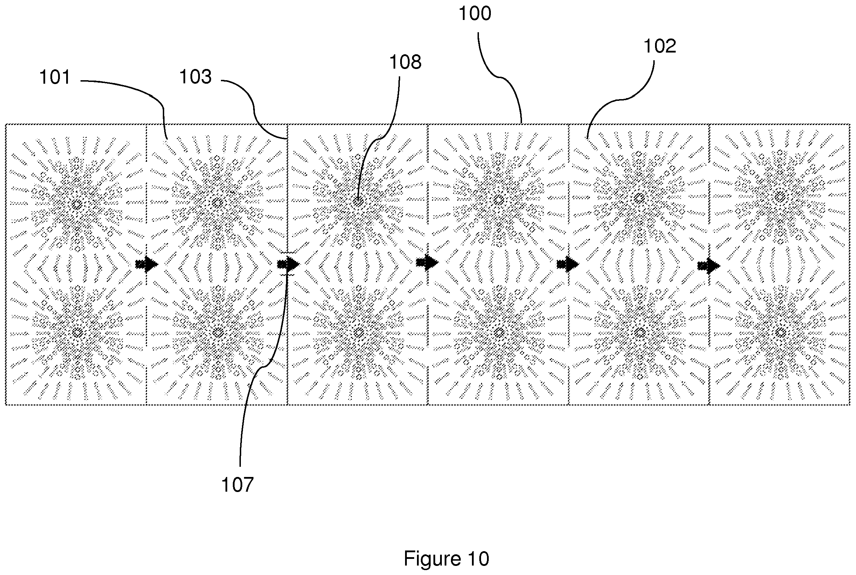

Chaque couple de cheminées est séparé d'un autre couple de cheminées par une distance déterminée 213 (figure 8). Dans la largeur, le secteur est muni d'au moins deux cheminées disposées à une distance donnée de la paroi, paroi engendrant une perte de charge et modifiant la portée du mouvement convectif. La perte de charge nécessite un rapprochement des cheminées des parois afin d'éviter que des zones ne soient pas suffisamment brassées. Ayant forcément deux parois canalisant le cheminement de la matière, il y a donc au moins deux cheminées. Dans le sens du déplacement de la matière (figure 10), la distance entre deux couples de cheminées est plus importante puisque l'expansion du gaz et le mouvement de matière ne sont pas limités par une paroi. Ce qui permet une expansion plus large du mouvement convectif de la matière. C'est ainsi, à titre d'exemple, que pour un couloir large de quatre mètres avec une hauteur de matière de cinq mètres, les deux cheminées sont disposées à 70 cm des parois et elles ont une distance de 2,6 mètres entre elle. La distance entre deux couples de cheminées est déterminée par l'évaluation du mouvement convectif et de sa modulation en fonction de la vitesse et du débit de gaz injecté. La distance séparant deux couples de cheminées est par exemple de trois mètres, le mouvement convectif de chaque couple de cheminées étant d'un rayon de 1,5 mètres, mouvement modulé et maîtrisé par la vitesse, le débit de gaz à l'embouchure des cheminées, vitesse et débit se modulant en fonction de la viscosité de la matière, viscosité renseignée par les variations de durée d'une injection donnée. Ces variations indiquent la viscosité dynamique de la matière en fonction des paramètres physique et biochimique de la matière dans le processus de fermentation.

La vitesse et le débit de gaz injecté dans les cheminées dépend de la largeur du front de matière, largeur variable dans un digesteur cylindrique canalisé par au moins deux parois, une paroi transversale divisant le flux de matière en deux et une paroi verticale délimitant un premier compartiment séparant à la fois la matière et le ciel gazeux du deuxième compartiment, laissant passer la matière par une large ouverture en partie basse de la paroi verticale.

Certaines cheminées interviennent séparément, notamment celles qui se situent dans un front réduit ou à proximité d'un passage plus étroit de matière en fermentation. Ces cheminées ont un impact sur la zone de brassage de matière plus limité, prenant en compte la proximité des parois et la perte de charge qui l'accompagne.

Comme illustré dans la figure 8, grâce à la poussée d'Archimède qui s'exerce sur un volume et un débit de gaz important autour de deux cheminées 201, la vitesse et le débit déterminent la largeur du brassage d'un secteur donné en fonction la viscosité de la matière.

L'injection est modulable en fonction de la viscosité de la matière. Le brassage d'un secteur par l'injection de gaz provenant d'un caisson ayant un volume déterminé, l'injection présentant un débit et une vitesse, est modulé par le niveau de pression duquel le gaz est détendu, en fonction de la viscosité de la matière en fermentation, viscosité renseignée par la durée de l'injection. Plus la viscosité est importante, plus la durée d'une injection de gaz est longue.

L'injection de 30 m³ de gaz avec une pression décroissante, dans le caisson, entre sept et quatre bars par exemple, dans deux cheminée est de l'ordre de 17 secondes dans le premier secteur du premier compartiment mais atteint 12 secondes dans le dernier secteur du deuxième compartiment.

En fonction de la viscosité de la matière, dépendant du secteur de fermentation, la vitesse et le débit du gaz injecté à partir du caisson de pression sont modulés. Par exemple, la pression du gaz dans le caisson de 10 m3 est montée à 7bars pour être détendue de 7 à 4 bars soit 30 Nm³ de biogaz dans le premier secteur du premier compartiment où la viscosité de la matière est forte, alors que la pression du gaz est montée à 6 bars, pour être détendue de 6 à 3 bars soit 30 Nm³ de biogaz dans le dernier secteur du deuxième compartiment où la viscosité de la matière a fortement diminuée sous l'action de l'hydrolyse et de la fermentation.

La modulation est favorable au processus de fermentation. Avec une vitesse et un débit insuffisants, le brassage et le mouvement convectif de la matière en fermentation dans un secteur donné serait insuffisants et engendreraient des passages préférentiels de la matière s'écoulant et des zones mortes de matière ne pouvant s'écouler. Avec une vitesse et un débit trop importants, on constaterait des zones de chevauchement importantes d'un secteur à l'autre. Des particules seraient emportées d'un secteur à l'autre à chaque cycle de brassage pour sortir rapidement de la cuve de fermentation, engendrant ainsi une durée de séjour très courte de certaines particules et une durée de séjour très longue pour d'autres.

Dans des modes de réalisation, grâce également à une première paroi interne à l'enceinte partant du plafond de l'enceinte, on crée deux compartiments dont les ciels gazeux sont séparés. Cette paroi laisse un passage en partie basse pour le passage de la matière d'un premier compartiment, où s'effectue l'hydrolyseacidogène vers le deuxième compartiment, où s'effectue l'acétogènèseméthanogénèse.

L'agitation sectorielle en continu en matière épaisse permet de réaliser dans au moins deux compartiments une dégradation importante de la matière organique, dans un processus permettant une hydrolyse importante de la matière à digérer et le respect des populations bactériennes dans chacune des phases de la fermentation, populations se développant dans des milieux biochimiques différents (premier compartiment à pH acide et température hyper-thermophile, deuxième compartiment à pH neutre ou légèrement basique et température thermophile ou mésophile), en produisant et en consommant des métabolites différents - acides gras volatils, éthanol, H₂, CO₂, Acétates, CH₄ etc..

La méthanisation est optimisée dans un dispositif fait de deux compartiments permettant que les différentes phases de la fermentation d'une matière épaisse se déroulent dans des milieux biochimiques favorables aux processus de dégradation de la matière. Notamment, l'hydrolyse des substrats entrants se déroule dans un milieu acide et une température hyper thermophile, permettant la simplification des molécules de matière organique complexe rendues disponibles à l'activité des bactéries acidogènes. Les bactéries acidogènes plus résistantes que les bactéries méthanogènes se développent favorablement à pH acide.

Le premier compartiment, d'hydrolyse-acidogène, permet de solubiliser les molécules complexes pour les rendre disponible aux bactéries acidogènes qui produisent, notamment des acides gras volatils, de l'hydrogène (H₂) et du gaz carbonique (CO₂).

Le rapport carbone/azote est préférentiellement de l'ordre de 15 à 25 pour un bon équilibre de la fermentation. Il dépend de la disponibilité réelle du carbone, notamment du carbone piégé dans la lignine. Un rapport faible risque de provoquer une augmentation d'ammoniac pouvant être toxique. Avec un pH élevé, par exemple entre 7,4 et 7,6, l'azote minéralisé sous forme d'ions ammonium se transforme pour partie en ammoniac plus volatil. A forte concentration, l'ammoniac devient inhibiteur de la méthanogénèse. La perte d'azote est un manque pour la valorisation agricole de la matière digérée, aussi appelée « digestat ». D'où l'intérêt d'un pilotage précis des phases de la fermentation, notamment d'une bonne hydrolyse rendant le carbone disponible favorisant un meilleure équilibre du rapport carbone/azote et, ainsi, une bonne maîtrise du pH dans une plage allant de 6,8 à 7,4 dans le deuxième compartiment, plage peu favorable au passage de l'ammonium à la forme ammoniac. Cet équilibre permet une meilleure conservation de l'azote.

Dans le domaine de la fermentation anaérobie en continu de substrat à de forts taux de matière sèche, il est connu de multiples formes de digesteurs, cylindriques, parallélépipédique, en couloir, multi-étagés qui utilisent soit des moyens mécaniques de transfert de la matière ou des moyens de brassage de la matière au biogaz sous pression. La présente invention ne se limite à aucune de ces formes.

La présente invention répond ainsi aux limites rencontrées dans les procédés anaérobies, en continu, à forte concentration en matière. Elle fournit, dans des modes de réalisation, un procédé qui consiste en une installation de fermentation anaérobie, continue, à forte concentration en matière sèche, multi phases, de mono et poly-substrat, en matière épaisse dans un seul fermenteur permettant la maîtrise de l'équilibre des phases d'hydrolyse, acidogène et méthanogène et du temps de séjour différencié de la matière selon la nature des substrats, par la progression régulière et l'écoulement d'un substrat épais et homogène à fort taux de matière sèche, de 15% à plus de 30% de matière sèche.

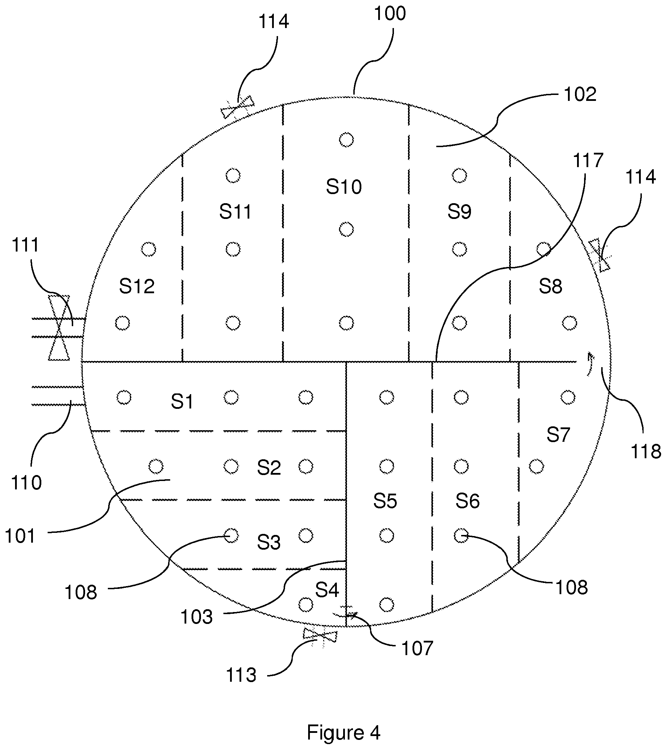

En ce qui concerne l'écoulement de type piston, une deuxième paroi de canalisation de la matière (117 en figure 4), dont la forme dépend de la géométrie de l'enceinte de fermentation, permet de réduire le front de matière, en favorisant une avancée de matière régulière.

Le brassage modulé par secteur de la matière tout au long du processus de fermentation permet l'extraction de la matière en fermentation et à sa re-circulation à travers les cheminées comme inoculum bactérien et régulateur biochimique d'un secteur à l'autre. Cet inoculum peut être extrait en différents points du fermenteur dans une sortie de diamètre suffisamment large pour permettre le passage et l'écoulement d'une matière plus ou moins hydrolysée et fermentée et donc plus ou moins épaisse, par gravité, pour être reprise par une pompe à matière épaisse et être réintroduite d'un secteur donné dans un autre secteur par une cheminée dotée d'une vanne gaz et d'une vanne matière, le plus souvent en amont. Cette re-circulation, si besoin, a pour fonction de renforcer l'activité bactérienne par réintroduction, dans un secteur en amont, de bactéries produites dans un secteur aval ou pour le rééquilibrage biochimique d'un secteur à l'autre.

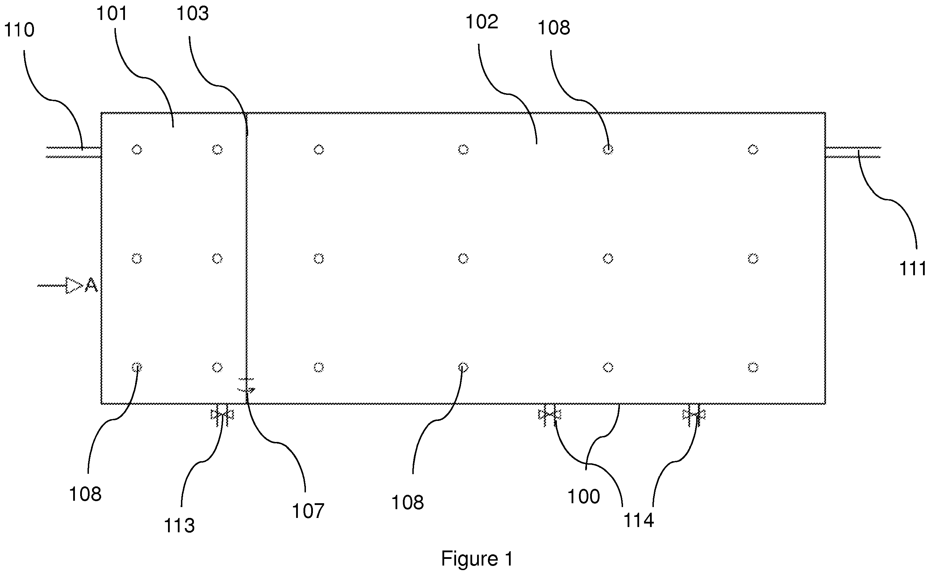

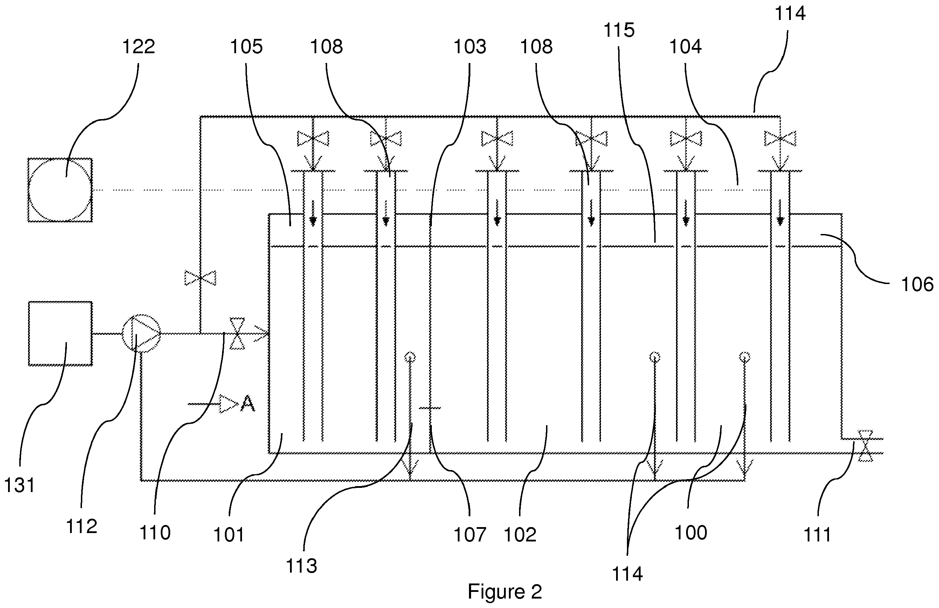

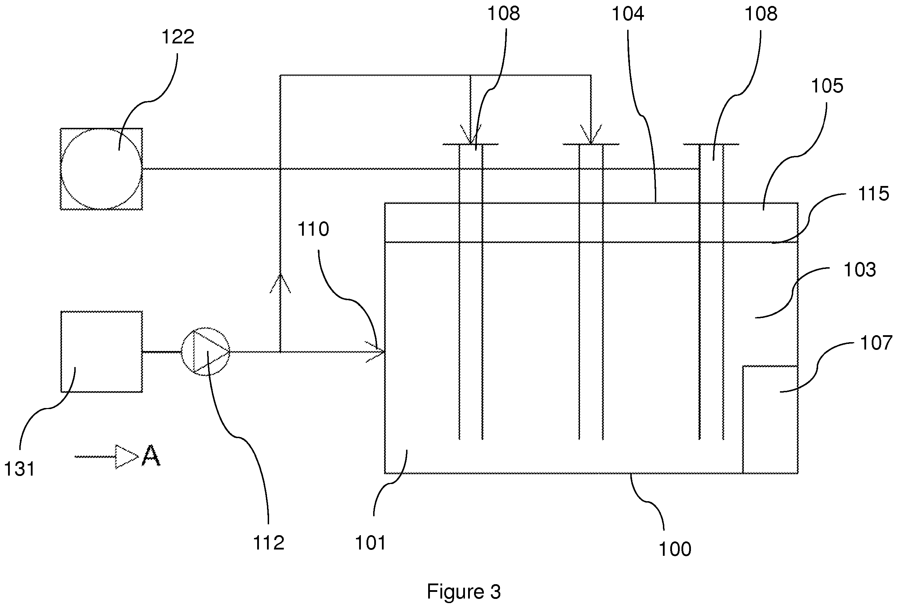

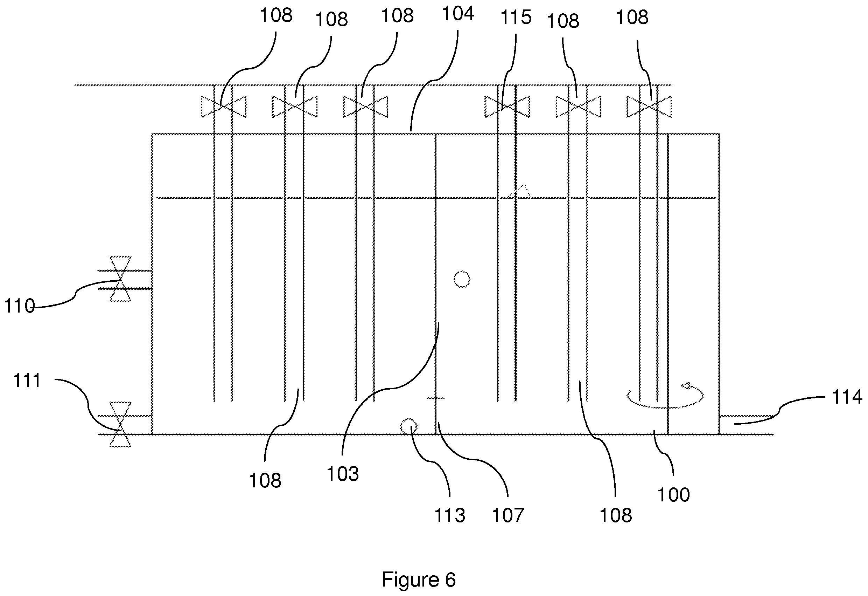

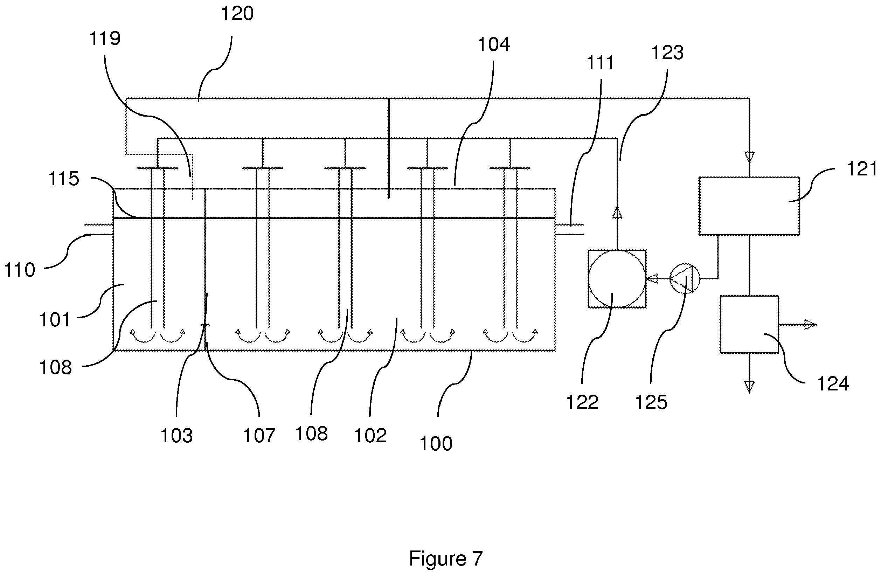

On observe, en figures 1 à 7 et 10, une enceinte de fermentation 100, un premier compartiment 101, un deuxième compartiment 102, une paroi 103 de séparation de matière, un plafond 104 du fermenteur, un ciel gazeux 105 du premier compartiment 101, un ciel gazeux 106 du deuxième compartiment 102, un passage de matière 107 entre le premier compartiment 101 et le deuxième compartiment 102. Par exemple, le passage 107 mesure 50 cm x 50 cm.

On observe aussi, dans les figures 1 à 7 et 10, des cheminées 108 d'injection de gaz, une entrée 110 de matière dans l'enceinte 101 du fermenteur, une sortie 111 de matière depuis l'enceinte 100 du fermenteur, un système d'introduction de la matière épaisse (par une pompe par exemple) 112, une tuyauterie de re-circulation de matière acide 113, une tuyauterie de re-circulation matière en fermentation 114, un niveau de matière 115, une cloison 117 de cheminement de matière, un passage de matière 118, une sortie 119 de gaz des ciels gazeux des compartiments du fermenteur, une tuyauterie 120 de gaz vers un gazomètre 121 à pression normale, un caisson 122 sous pression, des tuyaux 123 de gaz sous pression, un groupe de cogénération 124, un surpresseur compresseur 125, un mélangeur 131.

On observe, en figure 8, dans une enceinte 200, des cheminées 201, un plafond de fermenteur 202, un tuyau de gaz en pression 205, des vannes de gaz 204, un ciel gazeux 206 un niveau de matière avant injection 207, un niveau de refoulement de gaz 212 un fond 213 du fermenteur et un profil de mélange de surface 214.

Le dispositif illustré dans ces figures met notamment en œuvre une pluralité de cheminées 108 et 201 réparties dans l'ensemble du fermenteur, de diamètre suffisamment large, par exemple entre 10 et 15 cm de diamètre, partant du plafond du fermenteur et s'arrêtant à moins de 15 cm du fond du fermenteur, par exemple.

Les cheminées 108 et 201 sont répartie sur l'ensemble du fermenteur à raison d'une cheminé pour 4 à 10 m² de surface plancher, par exemple, selon le volume et la géométrie du fermenteur. L'espacement entre les cheminées 108 et 201 est fonction du cône de brassage engendré par le gaz injectée à une la pression de 4 à 10 bars, par exemple, par une tuyauterie 205 suffisamment large, de diamètre proche de celui des cheminées, afin de favoriser un débit et une vitesse de gaz arrivant au bas de la cheminée suffisamment élevés pour assurer le brassage du secteur à homogénéiser. Le débit et la vitesse dépendent de la surface du secteur à homogénéiser, du volume de matière, de la hauteur de matière dans le fermenteur et de l'interaction souhaitée avec les autres secteurs proches. Certaines cheminées peuvent être avantageusement situées au bord des parois et des cloisons du fermenteur pour compenser les éventuelles difficultés d'homogénéisation dues à l'effet de parois et des cloisons.

Le gaz en pression à plusieurs bars provenant d'un stockage de gaz dans un caisson en pression 122 alimente les cheminées 108 et 201 par un conduit de gaz 205.

Comme illustré en figure 8, par la poussée d'Archimède, le gaz exerce une poussée ascendante sur la matière autour de la cheminée 201, qui s'accompagne de bulles de gaz de fortes dimensions créant un mouvement convectif. La différence importante de masse volumique entre le gaz et la matière entraîne un mouvement de convection de la matière dans une zone plus large que la zone d'influence directe du gaz, dans la zone 210.

Un volume important de matière est déplacé par poussée ascendante grâce aux bulles de gaz qui remontent, créant ainsi un mouvement convectif de la matière amorçant un mouvement descendant dans une zone plus large par aspiration de la matière en partie basse autour de la cheminée 201. Ce mouvement convectif gaz-matière dépend du débit et de la pression du gaz injectée à plusieurs bars par le haut le plus souvent d'au moins un couple de cheminées.

Le gaz arrivant près du plancher du fermenteur exerce une poussée latérale qui se fait ressentir sur la surface tronqué du cône d'agitation d'un rayon de 0,5 à 1,5 mètres par exemple autour de la cheminée 201, en créant également un effet de balayage du fond, comme illustré en figure 8. Ce brassage est accentué par la synergie tourbillonnaire créée par l'injection de gaz sous pression dans plusieurs cheminées 201 proches activées en même temps. Ce mouvement entraîne également un gonflement de la matière et des vagues de surface, particulièrement efficaces et évitant toute production de croûte créée par des matières fibreuses, telles que de la paille.

Le volume de gaz comprimé dans le caisson 122, de dix à trente m³, par exemple, entre quatre et dix bars, par exemple, est fonction du volume du fermenteur, et des paramètres d'agitation. Le volume de gaz libéré du caisson peut être variable et passer par exemple, lors d'une injection de gaz, de six à quatre bars ou de sept à cinq bars en fonction du volume, de la vitesse de gaz souhaité, de la hauteur de matière dans le fermenteur, et de la viscosité de celle-ci.

Chaque secteur est activé successivement dès que la séparation des phases liquide solide est engagée dans le fermenteur. Cette séparation des matières solide et liquide est très lente dans un milieu épais à forte concentration en matière sèche. Un cycle complet de brassage, sur l'ensemble du fermenteur, est de plusieurs heures et peut être activé plusieurs fois par jour, plus ou moins longtemps en fonction de la de la viscosité de la matière dans le secteur concerné.

La compression séquentielle du gaz grâce à un surpresseur ou compresseur 125 dans le caisson 122 contenant le gaz sous pression, durant une période suffisamment longue (plusieurs dizaine de minutes), entre deux instants de libération du gaz sous pression dans le fermenteur, entre dix et vingt secondes, par exemple, dans un secteur défini par des cheminées 108 ou 201, permet de réduire considérablement la puissance du compresseur 125 dans un rapport de 100 environ.

Chaque secteur est déterminé par au moins deux cheminées recevant le gaz sous pression provenant du caisson 122. Chaque cheminée 108 ou 201 d'un secteur à brasser peut être activée conjointement aux autres cheminées 108 ou 201 du même secteur et recevoir du gaz en pression à partir d'une tuyauterie de gaz 112 et 205 alimentant plusieurs cheminées 108 ou 201 à la fois.

Le gaz injecté dans la ou les cheminées 108 ou 201, ressort dans le ciel gazeux du fermenteur pour se transférer naturellement par une canalisation 212 dans un réservoir souple ou gazomètre 121. Puis, une partie du gaz présent dans le gazomètre 121 est repris par un compresseur 125 qui comprime le gaz dans le caisson 122 avant une nouvelle injection dans un nouveau secteur.

Certaine cheminées 108 permettent une re-circulation de matière, en fermentation ou fermentée en des points différenciés pour inoculer des bactéries ou équilibrer biochimiquement certains secteurs.

L'introduction et la re-circulation de matière se font à l'aide d'une pompe à matière épaisse 112, par exemple de type pompe à vis ou à béton ou vis sans fin. La re-circulation de la matière en fermentation se fait à partir de tuyauteries de matière 113 et 114, par exemple, ce qui permet de recycler une matière acide en fermentation 113 ou une matière méthanogène en fermentation 114. Le dispositif comporte également une première paroi 103 séparant deux compartiments 101 et 102 dans l'enceinte 100 du fermenteur. Cette paroi 103 monte jusqu'au plafond de l'enceinte 100 du fermenteur, rendant les ciels gazeux 105 et 106 des compartiments 101 et 102 indépendantes. La paroi 103 est ouverte en partie basse par le passage 107 de la matière en fermentation du premier compartiment au deuxième compartiment. Grâce à l'hydrolyse et l'homogénéisation due au brassage, le passage de la matière se fait par simple gravité engendrée par l'apport d'une matière nouvelle, selon la loi de l'hydrostatique des fluides (équilibre des niveaux), après brassage si besoin, des secteurs acidogènes du premier compartiment 101.

Cette paroi 103 permet d'avoir, dans la même enceinte 100 du fermenteur, deux niveaux de température différents, de pouvoir fonctionner dans le premier compartiment, en hydrolyse thermo-enzymatique, de l'ordre de 65°C, et dans le deuxième compartiment en mésophile, de 38 à 40°C, par exemple, voir thermophile. Le premier compartiment 101, d'hydrolyse-acidogène, permet de solubiliser les molécules complexes pour les rendre disponible aux bactéries acidogènes qui produisent, notamment des acides gras volatils, de l'éthanol, de l'Hydrogène et du gaz carbonique. Le pH du milieu diminue entre 5 à 6 par exemple, sans descendre en dessous de 4,5, selon les substrats, avant la phase de méthanogènes du deuxième compartiment où le pH est ramené à un niveau proche de la neutralité grâce à l'action des différentes familles de bactéries méthanogènes transformant les acides gras volatils, l'hydrogène et le gaz carbonique en biogaz formé essentiellement de méthane et de gaz carbonique et minéralisant l'azote.

Le pilotage de la fermentation à partir du taux de pH et le taux d'H₂ dans les ciels gazeux et en phase soluble dans le substrat (pression partielle d'H₂), dans chaque compartiment est rendu possible par la re-circulation différenciée de la matière en fermentation ou fermentée en différents endroits du fermenteur par le biais des cheminées. Ce pilotage des niveaux de pH et d'H₂ conforme à l'activité bactérienne des différentes familles de bactéries favorise la vitesse et le taux de dégradation de la matière organique en CH₄ et CO₂ et la réduction du temps de séjour de la matière dans le fermenteur. L'hydrolyse thermo-enzymatique permet au carbone piégé dans la lignine de devenir disponible pour la fermentation acidogène et méthanogène et de favoriser un meilleur équilibre carbone azote, notamment dans les fumiers pailleux. La cloison 103 permet ainsi de maîtriser séparément les phases d'hydrolyse-acidogène de la méthanogénèse

Les deux niveaux de température différents sont gérés par un système d'échangeurs thermiques apportant les calories au fonctionnement hyper-thermophile du premier compartiment 101 et pouvant extraire les calories en surplus dans le deuxième compartiment 102 dû à l'apport d'une matière en fermentation dotée d'une température plus élevée venant du premier compartiment 101. Cependant l'excédent de température du premier compartiment 101 peut suffire pour compenser les pertes d'énergie à travers les parois du fermenteur sur l'ensemble de celui-ci.

Le premier compartiment 101 est muni d'une voie d'extraction de la matière juste avant le passage de la matière acidogène 113 afin de pouvoir re-circuler une partie de la biomasse du substrat acide dans le mélangeur 131 avec le substrat nouveau ou directement dans une des cheminée 108 à l'aide de la pompe en matière épaisse 112.

La paroi 103 détermine le volume du premier compartiment qui représente entre un sixième et un quart du volume total du fermenteur, par exemple. Dans une variante, la paroi 103 s'arrête à quelques dizaines de centimètres, par exemple, du fond du fermenteur laissant un passage sur la totalité du bas de la paroi. Cette cloison est adaptée en fonction de la géométrie du fermenteur. Dans une autre variante le passage 117 fait l'objet d'un système d'écluse pouvant fermer ponctuellement le passage entre les compartiments 101 et 102. La fermeture peut être actionnée de l'extérieur du fermenteur par l'introduction, dans une fente disposée sur le fermenteur, d'une plaque bouchant le passage. Ce système d'écluse, ne devant être actionné qu'au moment du démarrage du fermenteur ou en cas d'introduction accidentelle d'une matière toxique de la fermentation, peut être également mécanisé.

Le fermenteur comporte, en fonction de la taille et de la géométrie du fermenteur, une deuxième paroi 117 de canalisation de la matière dans son cheminement d'un point d'entrée dans le fermenteur jusqu'à son extraction du fermenteur en fonction du temps de séjour. Le front de matière est ainsi réduit et le cheminement allongé par un facteur environ de deux. Le front de matière se déplace du point d'introduction 110 au point d'extraction 111. Ce qui permet également de ne pas mélanger les principales populations bactériennes qui interviennent à un stade déterminé du processus et n'entrent pas ainsi en compétition. Cette deuxième paroi 117 monte jusqu'au sommet du fermenteur dans le premier compartiment 101, séparant les ciels gazeux des deux compartiments et ne monte pas jusqu'au plafond dans le deuxième compartiment 102, laissant ainsi un ciel gazeux entièrement libre de chaque côté de la paroi 117.

Cette variante est particulièrement adaptée pour des installations de grandes capacités et pour des fermenteurs cylindriques. Cette paroi 117 peut être supprimée pour des fermenteurs rectangulaires où la longueur est beaucoup plus importante que la largeur.

La figure 4 présente une vue de dessus du fermenteur cylindrique séparé en deux compartiments 101 et 102 séparé par une paroi 103 déterminant deux atmosphères 105 et 106, et une cloison 117 canalisant la matière sur le deuxième compartiment 102 et laissant un passage 118 pour permettre le passage de la matière sur la deuxième partie du deuxième compartiment 102.

Les cheminées 108 organisées par quatre, secteur S5, trois, secteurs S1, S2, S4, S6, S7, S10, S11, S12, deux, secteurs S3, S8, S9, S13 ou par unité près des zones de passage en partie basse de la paroi de séparation 103, secteurs S4, S5 déterminent des secteurs d'agitation dont l'agitation est à maîtriser aussi bien sur la hauteur de matière que sur la largeur du plancher du dit secteur par la modulation du brassage lui-même par la vitesse et un débit variable, dépendant du niveau de pression dans le caisson 122, le niveau de pression dépendant de la durée de la compression, 8,5 minutes de compression pour monter à six bars relatifs et dix minutes de compression pour monter à sept bars relatifs. Le gaz est alors détendu en 15 secondes par exemple de six à quatre bars ou de sept à cinq bars, ce qui fait varier la vitesse et le débit du gaz à l'embouchure d'une cheminée en fonction du nombre de cheminées. La vitesse et le débit peuvent être modulé à volonté et s'ajuster à la viscosité de la matière en fermentation.

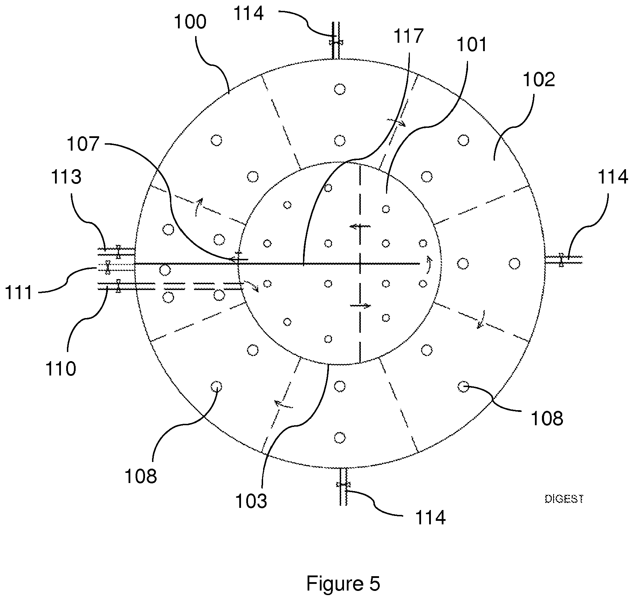

La figure 5 présente un dispositif de fermenteur cylindrique notamment adapté à des volumes de fermenteur important.

Le dispositif repose sur deux compartiments 101 et 102 dans une seule enceinte 100. Le premier compartiment d'hydrolyse thermo-enzymatique et d'acidogénèse 101 est situé à l'intérieur dans une enceinte 103 qui tient lieu de parois entre les deux compartiments 101 et 102. La matière est introduite par une pompe à matière épaisse dans le premier secteur S1 à travers une cheminée 108 ou directement dans la cuve par une canalisation reliée à la pompe à matière épaisse 122. La matière brassée par secteur par plusieurs cheminées 108, s'hydrolysant grâce à la température hyper-thermophile se déplace dans les secteurs S1 à S4, autour de la paroi de canalisation de la matière 117 pour sortir par un large passage 107 pour poursuivre son cheminement par l'écoulement dans les secteurs S5 à S13 grâce au brassage à fort débit par deux cheminées dans un écoulement piston, écoulement rendue plus facile par la solubilisation progressive de la matière pour sortir du fermenteur sous la forme d'un digestat restant épais en 111.

Le dispositif est également pourvu d'un pilotage modélisé et plus ou moins automatisé selon le type d'installation. Ce pilotage active, de façon différenciée, les vitesses et débits de gaz à injecter dans les cheminées à partir du niveau de pression dans le caisson à l'ouverture de l'injection et du niveau bas de fin d'injection, en fonction de la viscosité de la matière renseignée par la durée de brassage à volume, vitesse et débit de gaz donnée. Ce pilotage active également l'ouverture ou la fermeture des vannes de gaz ou de matière et les quantités de re-circulation de la matière en des points déterminés du fermenteur par les cheminées 108 ou 201, en fonction des paramètres d'équilibre du fermenteur (qualité du gaz, pH ....) et des caractéristiques des substrats à fermenter. Ce pilotage d'un ensemble de vannes et de gaz ou de matière, plus ou moins automatisé, permet de moduler les quantités à re-circuler en des endroits différenciés du fermenteur en fonction des indicateurs de gaz ou matière qui peuvent être suivis dans les deux compartiments 101 et 102 pour le gaz et à partir des cheminées 108 ou 201, pour la matière en tout endroit du fermenteur. Les indicateurs de pilotage peuvent être téléchargés sur un serveur central qui renvoie les consignes de fonctionnement et de pilotage du fermenteur.

Le procédé de méthanisation continue en voie sèche, dans un fermenteur comportant une cuve fermée comporte :
- une étape d'introduction de matière épaisse à fermenter comportant au moins 15 % de matière sèche, dans au moins un compartiment de ladite cuve et
- une étape d'injection, par l'intermédiaire d'au moins une cheminée qui descend à travers au moins un dit compartiment, de gaz sous pression à proximité du fond du compartiment, de manière à créer, par la remontée du gaz injecté à travers la matière épaisse, un mouvement ascendant dans la matière épaisse autour de la cheminée, en brassant la matière se trouvant au fond du compartiment.

Comme exposé ci-dessus, on introduit la matière épaisse dans un premier compartiment et, après hydrolyse dans le premier compartiment, on fait passer la matière épaisse hydrolysée dans un deuxième compartiment où a lieu la méthanogénèse. L'étape de passage du premier au deuxième compartiment est effectuée par le fond de la cuve, les atmosphères des compartiments n'étant ainsi pas en contact.

Préférentiellement, au cours de l'injection de gaz, on introduit du gaz issu de la fermentation de la matière épaisse et on injecte du gaz dans au moins deux cheminées proches.

Le procédé comporte, préférentiellement, une étape de mesure de viscosité de la matière épaisse, dans lequel, au cours de l'étape d'injection de gaz, la quantité de gaz, son débit et/ou sa vitesse sont fonction de la viscosité mesurée. Par exemple au cours de l'étape de mesure de viscosité de la matière épaisse, on mesure la résistance de la matière épaisse à l'injection de gaz lors de l'injection de gaz. Plus particulièrement, au cours de l'étape de mesure de viscosité de la matière épaisse, on mesure une durée d'une baisse de pression d'un volume prédéterminée du gaz injecté.

Préférentiellement, le fermenteur comporte une pluralité de cheminées, dans lequel, au cours de l'étape d'injection de gaz, on injecte du gaz successivement dans les cheminées. Au cours de l'étape d'injection de gaz, on injecte du gaz successivement dans les cheminées allant de l'amont vers l'aval du chemin suivi par la matière épaisse.

Préférentiellement, au cours de l'étape d'injection de gaz, on injecte, dans la matière épaisse, un gaz sous une pression au moins cinq à dix fois supérieure à la pression de la hauteur de la colonne de matière autour de la cheminée. Préférentiellement, le procédé comporte alternativement :
- une étape d'injection du gaz dans la matière épaisse et
- une étape de mise en compression de biogaz dans un caisson de stockage en pression.

Par exemple, le rapport cyclique entre la durée d'injection de gaz et la durée de mise en compression inférieur à un trentième, par exemple entre un cinquantième et un centième.

Dans des modes de réalisation, le procédé comporte une étape de prélèvement de matière épaisse sur le chemin suivi par la matière épaisse et une étape d'injection de la matière épaisse en amont du point de prélèvement.

## Revendications

1. Procédé de méthanisation continue en voie sèche, dans un fermenteur comportant une cuve fermée (100, 200), qui comporte de fermenter de la matière épaisse comportant au moins 15 % de matière sèche, dans au moins un compartiment (101, 102) de ladite cuve, **caractérisé en ce qu'**il comporte, de plus, d'injecter, par l'intermédiaire d'une pluralité de cheminée (108, 201), chaque cheminée descendant à travers au moins un dit compartiment, du biogaz sous pression à proximité du fond du compartiment, de manière à créer, par la remontée (209) du biogaz injecté à travers la matière épaisse, un mouvement convectif, en brassant notamment la matière se trouvant au fond du compartiment ; ledit biogaz est injecté dans au moins deux cheminées proches de sorte à réaliser des fronts de brassage qui sont favorables au déplacement en translation de la matière en cours de fermentation, dans lequel le fermenteur comporte une pluralité de cheminées et dans lequel, au cours de l'étape d'injection de biogaz, on injecte du biogaz successivement dans les cheminées allant de l'amont vers l'aval du chemin suivi par la matière épaisse.

2. Procédé selon la revendication 1, dans lequel on hydrolyse de la matière épaisse à au moins 15 % dans un premier compartiment (101) et, la matière épaisse hydrolysée s'écoule dans un deuxième compartiment (102) où a lieu la méthanogenèse.

3. Procédé selon la revendication 2, dans lequel l'étape de passage du premier compartiment (101) au deuxième compartiment (102) est effectuée par le fond (107, 118) de la cuve (100), les atmosphères des compartiments n'étant ainsi pas en contact.

4. Procédé selon l'une des revendications 1 à 3, dans lequel, au cours de l'injection de biogaz, on introduit du biogaz issu de la fermentation de la matière épaisse.

5. Procédé selon l'une des revendications 1 à 4, qui comporte une étape de mesure de viscosité de la matière épaisse, dans lequel, au cours de l'étape d'injection de biogaz, la quantité de biogaz, son débit et/ou sa vitesse sont fonction de la viscosité.

6. Procédé selon la revendication 5, dans lequel, au cours de l'étape de mesure de viscosité de la matière épaisse, on mesure la résistance de la matière épaisse à l'injection de biogaz lors de l'injection de biogaz.

7. Procédé selon l'une de revendications 1 à 6, qui comporte une étape de prélèvement de matière épaisse sur le chemin suivi par la matière épaisse et une étape d'injection de la matière épaisse en amont du point de prélèvement.

8. Procédé selon l'une des revendications 1 à 7, dans lequel au cours de l'étape d'injection de biogaz, on injecte, dans la matière épaisse, un biogaz sous une pression au moins cinq fois supérieure à la pression de la hauteur de la colonne de matière autour de la cheminée et au minimum 4 bar relatif.

9. Dispositif de méthanisation continue en voie sèche, dans un fermenteur comportant une cuve fermée (100), qui comporte un moyen d'introduction de matière épaisse à fermenter comportant au moins 15 % de matière sèche, dans au moins un compartiment (101, 102) de ladite cuve,
**caractérisé en ce qu'**il comporte, de plus :
- un moyen d'injection, par l'intermédiaire d'une pluralité de cheminées (108, 201), chaque cheminée descendant à travers au moins un dit compartiment, de biogaz sous pression à proximité du fond du compartiment, pour créer, par la remontée (209) du biogaz injecté à travers la matière épaisse, un mouvement convectif dans la matière épaisse autour de la cheminée, en brassant la matière se trouvant notamment au fond du compartiment,
- un moyen de commande de l'injection de biogaz dans chacune des dites cheminées, pour injecter du biogaz dans au moins deux cheminées proches, dans lequel le moyen de commande de l'injection de biogaz commande une injection du biogaz successivement dans les cheminées allant de l'amont vers l'aval du chemin suivi par la matière épaisse.

10. Dispositif selon la revendication 9, dans lequel la cuve (100) comporte un premier compartiment (101) dans lequel on introduit la matière épaisse et un deuxième compartiment (102) dans lequel, après hydrolyse dans le premier compartiment, la matière épaisse hydrolysée s'écoule, la méthanogenèse se réalisant dans le deuxième compartiment.

11. Dispositif selon la revendication 10, qui comporte un passage (107, 118) par le fond de la cuve (100) entre le premier compartiment (101) et le deuxième compartiment (102), les atmosphères des compartiments n'étant ainsi pas en contact.

12. Dispositif selon l'une des revendications 9 à 11, dans lequel le moyen d'injection de biogaz est configuré pour introduire du biogaz issu de la fermentation de la matière épaisse.

13. Dispositif selon l'une des revendications 9 à 12, qui comporte un moyen de mesure de viscosité de la matière épaisse, le moyen d'injection de biogaz étant configuré pour que la quantité de biogaz injecté, son débit et/ou sa vitesse soient fonction de la viscosité mesurée.

14. Dispositif selon l'une de revendications 9 à 13, qui comporte un moyen de prélèvement (113, 114) de matière épaisse sur le chemin suivi par la matière épaisse et un moyen d'injection (108) de la matière épaisse en amont du point de prélèvement.

15. Dispositif selon l'une des revendications 9 à 14, dans lequel le moyen d'injection de biogaz est configuré pour injecter, dans la matière épaisse, un biogaz sous une pression au moins cinq fois supérieure à la pression de la hauteur de la colonne de matière autour de la cheminée et au minimum de 4 bars relatif.

16. Dispositif selon l'une des revendications 9 à 15, qui comporte un moyen de pilotage de l'ouverture ou de la fermeture des vannes de gaz ou de matière et dans lequel les quantités de re-circulation de la matière en des points déterminés du fermenteur par les cheminées (108, 201), sont déterminés en fonction des paramètres d'équilibre du fermenteur et des caractéristiques des substrats à fermenter

## Patentansprüche

1. Kontinuierliches Methanisationsverfahren auf dem Trockenweg, in einem ein geschlossenes Becken (100, 200) umfassenden Fermentierer, das das Fermentieren der wenigstens 15 % Trockenmasse umfassenden dickflüssigen Masse in wenigstens einer Kammer (101, 102) des genannten Beckens umfasst, **dadurch gekennzeichnet, dass** es darüber hinaus das Einschießen von mit Druck beaufschlagtem Biogas in der Nähe des Bodens der Kammer mittels einer Vielzahl von Schächten (108, 201), wobei jeder Schacht durch wenigstens eine der genannten Kammern herabführt, derart umfasst, dass durch das Wiederaufsteigen (209) des durch die dickflüssige Masse eingeschossenen Biogases eine konvektive Bewegung ausgelöst wird, indem insbesondere die sich am Boden der Kammer befindende Masse vermischt wird; wobei das genannte Biogas in wenigstens zwei nahe Schächte derart eingeschossen wird, dass Vermischungsfronten, die für die Verschiebung der sich in der Fermentierung befindenden Masse in Translation günstig sind, realisiert werden, bei der der Fermentierer eine Vielzahl von Schächten umfasst und bei der im Verlauf des Biogas-Einschießschritts Biogas sukzessive in die Schächte eingeschossen wird, die von dem Weg, der von der dickflüssigen Masse zurückgelegt wird, von vorne nach hinten zurückgelegt wird.

2. Verfahren gemäß Anspruch 1, bei dem die dickflüssige Masse zu wenigstens 15 % in einer ersten Kammer (101) hydrolisiert wird und die hydrolyiserte dickflüssige Masse in einer zweiten Kammer (102) abläuft, in der die Methanogenese stattfindet.

3. Verfahren gemäß Anspruch 2, bei dem der Durchgangsschritt von der ersten Kammer (101) in die zweite Kammer (102) durch den Boden (107, 108) des Beckens (100) durchgeführt wird, wobei die Atmosphären der Kammern somit nicht in Kontakt sind.

4. Verfahren gemäß einem der Ansprüche 1 bis 3, bei dem im Verlauf des Einschießens von Biogas aus der Fermentierung der dickflüssigen Masse stammendes Biogas eingeführt wird.

5. Verfahren gemäß einem der Ansprüche 1 bis 4, das einen Messschritt der Viskosität der dickflüssigen Masse umfasst, in der im Verlauf des Einschießschritts von Biogas die Biogasmenge, sein Durchsatz und / oder seine Geschwindigkeit von der Viskosität abhängen.

6. Verfahren gemäß Anspruch 5, bei dem im Verlauf des Messschritts der Viskosität der dickflüssigen Masse der Widerstand der dickflüssigen Masse beim Einschießen von Biogas beim Einschießen von Biogas gemessen wird.

7. Verfahren gemäß einem der Ansprüche 1 bis 6, das einen Entnahmeschritt von dickflüssiger Masse auf dem von der dickflüssigen Masse zurückgelegten Weg und einen Einschießschritt der dickflüssigen Masse vor dem Entnahmepunkt umfasst.

8. Verfahren gemäß einem der Ansprüche 1 bis 7, bei dem im Verlauf des Einschießschritts von Biogas ein Biogas mit einer Druckbeaufschlagung, die mindestens fünf Mal höher ist als der Druck der Höhe der Massensäule um den Schacht und mit mindestens 4 relativen bar in die dickflüssige Masse eingeschossen wird.

9. Kontinuierliche Methanisationsvorrichtung auf dem Trockenweg in einem ein geschlossenes Becken (100) umfassenden Fermentierer, die ein Einführmittel von zu fermentierender dickflüssiger Masse umfasst, umfassend wenigstens 15 % Trockenmasse, in wenigstens einer Kammer (101, 102) des genannten Beckens, **dadurch gekennzeichnet, dass** sie darüber hinaus umfasst:
- ein Einschießmittel von mit Druck beaufschlagtem Biogas in der Nähe des Bodens der Kammer mittels einer Vielzahl von Schächten (108, 201), wobei jeder Schacht durch wenigstens eine genannte Kammer herabführt, um durch das Wiederaufsteigen (209) des durch die dickflüssige Masse eingeschossenen Biogases durch Vermischen der sich insbesondere am Boden der Kammer befindenden Masse eine konvektive Bewegung in der dickflüssigen Masse um den Schacht auszulösen,
- ein Steuermittel des Einschießens von Biogas in jeden der genannten Schächte zum Einschießen des Biogases in wenigstens zwei nahe Schächte, in dem das Steuermittel des Einschießens von Biogas ein Einschießen des Biogases sukzessive in die Schächte steuert, die auf dem von der dickflüssigen Masse zurückgelegten Weg von vorn nach hinten führen.

10. Vorrichtung gemäß Anspruch 9, bei der das Becken (100) eine erste Kammer (101), in der die dickflüssige Masse eingeführt wird, und eine zweite Kammer (102), in der die hydroylisierte dickflüssige Masse nach der Hydrolyse in der ersten Kammer abläuft, wobei die Methanogenese in der zweiten Kammer erfolgt, umfasst.

11. Vorrichtung gemäß Anspruch 10, die einen Durchgang (107, 118)durch den Boden des Beckens (100) zwischen der ersten Kammer (101) und der zweiten Kammer (102) umfasst, wobei die Atmosphären der Kammern somit nicht in Kontakt sind.

12. Vorrichtung gemäß einem der Ansprüche 9 bis 11, bei der das Einschießmittel von Biogas zum Einführen des aus der Fermentierung der dickflüssigen Masse stammenden Biogases ausgestaltet ist.

13. Vorrichtung gemäß einem der Ansprüche 9 bis 12, die ein Messmittel der Viskosität der dickflüssigen Masse umfasst, wobei das Einschießmittel von Biogas ausgestaltet ist, damit die Menge an eingeschossenem Biogas, sein Durchsatz und / oder seine Geschwindigkeit von der gemessenen Viskosität abhängen.

14. Vorrichtung gemäß einem der Ansprüche 9 bis 13, die ein Entnahmemittel (113, 114) von dickflüssiger Masse auf dem von der dickflüssigen Masse zurückgelegten Weg und ein Einschießmittel (108) der dickflüssigen Masse vor dem Entnahmepunkt umfasst.

15. Vorrichtung gemäß einem der Ansprüche 9 bis 14, bei der das Einschießmittel von Biogas ausgestaltet ist, um ein Biogas mit einer Druckbeaufschlagung, die mindestens fünf Mal höher ist als der Druck der Höhe der Massensäule um den Schacht und mit mindestens 4 relativen bar in die dickflüssige Masse einzuschießen.

16. Vorrichtung gemäß einem der Ansprüche 9 bis 15, die ein Steuermittel der Öffnung oder der Schließung der Gas- oder Massenventile umfasst und bei der die Rücklaufmengen der Masse in bestimmten Punkten des Fermentierers durch die Schächte (108, 201) in Abhängigkeit von Ausgleichsparametern des Fermentierers und der Merkmale der zu fermentierenden Substrate bestimmt sind.

## Claims

1. Method of continuous dry methanation, in a fermenter comprising a closed tank (100, 200), that comprises fermenting slurry, comprising at least 15% dry matter, in at least one compartment (101, 102) of said tank, **characterized in that** it further comprises injecting pressurized gas, via a plurality of chimneys (108, 201), each chimney descending through at least one said compartment, pressurized gas close to the bottom of the compartment, configured to create, by the injected gas rising (209) through the slurry, a convective movement in the slurry around the chimney, stirring the matter that is found in particular at the bottom of the compartment; wherein gas is injected into at least two chimneys close to each other in order to produce stirring fronts that are favorable to the translation movement of the matter undergoing fermentation, wherein the fermenter comprises a plurality of chimneys and wherein, during the gas injection step, gas is injected successively into the chimneys going from upstream to downstream on the path followed by the slurry.

2. Method according to claim 1, wherein the slurry is hydrolyzed to at least 15% in a first compartment (101) and the hydrolyzed slurry flows into a second compartment (102) where methanogenesis takes place.

3. Method according to claim 2, wherein the step of passing from the first compartment (101) to the second compartment (102) is performed at the bottom (107, 118) of the tank (100), the atmospheres of the compartments thus not being in contact.

4. Method according to one of claims 1 to 3, wherein, during the gas injection, gas from the fermentation of the slurry is introduced.

5. Method according to one of claims 1 to 4, which comprises a step of measuring the viscosity of the slurry wherein, during the gas injection step, the quantity of gas, its flow rate and/or its speed are dependent upon the viscosity measured.

6. Method according to claim 5, wherein, during the step of measuring the viscosity of the slurry, the slurry's resistance to the injection of gas is measured during the injection of gas.

7. Method according to one of claims 1 to 6, which comprises a step of collecting slurry on the path followed by the slurry, and a step of injecting slurry upstream of the collection point.

8. Method according to one of claims 1 to 7, wherein during the gas injection step, a gas is injected into the slurry that has a pressure at least five times higher than the pressure at the height of the column of matter around the chimney, and at least 4 relative bars.

9. Device for continuous dry methanation, in a fermenter comprising a closed tank (100), that comprises a means of introducing slurry to be fermented, comprising at least 15% dry matter, into at least one compartment (101, 102) of said tank, characterized it further comprises:
- a means of injecting, via a plurality of chimneys (108, 201), each chimney descending through at least one said compartment, pressurized gas close to the bottom of the compartment, configured to create, by the injected gas rising (209) through the slurry, a convective movement in the slurry around the chimney, stirring the matter that is found in particular at the bottom of the compartment,
- a means of controlling the injection of gas into each of said chimneys, to inject gas into at least two chimneys close to each other, wherein the means of controlling the injection of gas controls an injection of gas successively into the chimneys going from upstream to downstream on the path followed by the slurry.

10. Device according to claim 9, wherein the tank (100) comprises a first compartment (101) into which the slurry is introduced and a second compartment (102) into which the hydrolyzed slurry flows after hydrolysis in the first compartment, methanogenesis taking place in the second compartment.

11. Device according to claim 10, which comprises a passage (107, 118) at the bottom of the tank (100) between the first compartment (101) and the second compartment (102), the atmospheres of the compartments thus not being in contact.

12. Device according to one of claims 9 to 11, wherein the means of injecting gas is configured to introduce gas from the fermentation of the slurry.

13. Device according to one of claims 9 to 12, which comprises a means of measuring the viscosity of the slurry, the means of controlling the injection of gas being configured such that the quantity of gas injected, its flow rate and/or its speed are dependent upon the viscosity measured.

14. Device according to one of claims 9 to 13, which comprises a means of collecting (113, 114) slurry on the path followed by the slurry, and a means of injecting (108) slurry upstream of the collection point.

15. Device according to one of claims 9 to 14, wherein the means of injecting gas is configured to inject a gas into the slurry that has a pressure at least five times higher than the pressure at the height of the column of matter around the chimney, and at least 4 relative bars.

16. Device according to one of claims 9 to 15, which comprises a actuating means that actuate the opening or closing of gas or matter valves, wherein the recirculated quantities of matter at defined points of the fermenter by chimneys (108, 201) are determined depending on the fermenter's balance parameters and on characteristics of the substrates to be fermented.
